# EUROPÄISCHE PATENTSCHRIFT

(11) **EP 2 570 092 B1**
(45) Veröffentlichungstag und Bekanntmachung des Hinweises auf die Patenterteilung: **22.06.2016**
(21) Anmeldenummer: 12184025.0
(22) Anmeldetag: 12.09.2012
(51) Int. Cl.: A61B 17/72

(54) **Marknagel**
Marrow nail
Clou médullaire

(30) Priorität: 15.09.2011 DE 102011053638
(43) Veröffentlichungstag der Anmeldung: 20.03.2013
(73) Patentinhaber: Wittenstein AG, 97999 Igersheim (DE)
(72) Erfinder: Stauch, Roman, 97959 Assamstadt (DE)
(74) Vertreter: Zimmermann & Partner Patentanwälte mbB

(56) Entgegenhaltungen:
- DE-A1- 10 317 776
- DE-C2- 19 708 279
- FR-A1- 2 949 662

## Beschreibung

### Gebiet der Erfindung

Die Erfindung betrifft einen Marknagel für eine Distraktion eines Röhrenknochens und ein Baukastensystem nach dem nebengeordneten Anspruch.

### Stand der Technik

Aus dem Stand der Technik sind Marknägel bekannt, welche eine Distraktion von langen Röhrenknochen ermöglichen. Zwei Knochenfragmente, ein distales Fragment und ein proximales Fragment, werden relativ zueinander mittels eines Marknagels verschoben. An der Kontaktstelle zwischen den beiden Knochenfragmenten soll Knochen nachwachsen. Dies wird erreicht, indem die Vorschubgeschwindigkeit des Marknagels zur Distraktion ausreichend klein gewählt wird.

Weiterhin ist aus dem Stand der Technik bekannt, Marknägel mit einer Möglichkeit zur Segmentverschiebung einzusetzen, beispielsweise sei hier der Stand der Technik aus der DE 197 08 279 C2 zitiert. Solche Segmentverschiebungen ermöglichen eine Behandlung von großen Knochendefekten von mehr als beispielsweise 3 cm, wie sie in Folge von Krankheiten oder Gewalteinwirkung entstehen können. Auch bei Operationen aufgrund von Knochentumoren können große Knochendefekte entstehen, welche je nach Schweregrad der Erkrankung mit einem Marknagel mit Segmentverschiebung behandelt werden können.

Eine Herausforderung bei Marknägeln mit Segmenttransport sind grundsätzlich die Fixierungen der Enden des Marknagels in dem proximalen Knochenfragment und dem distalen Knochenfragment. Eine weitere Herausforderung ist die Stabilisierung gegen Rotationen.

So schlägt die DE 197 08 279 C2 eine Führungshülse vor, welche mit einer in axialer Richtung des Röhrenknochens eingedrehten Schraube im Knochenfragment fixiert wird. Die Führungshülse als zusätzliches Bauteil macht den Einbau des Marknagels im Knochen kompliziert. Die Führungshülse benötigt außerdem einen zusätzlichen Freiraum im Knochenfragment, sodass der Durchmesser der Öffnung, welche im Knochenfragment geschaffen werden muss, groß ist. Weiterhin lässt sich der Marknagel des oben zitierten Standes der Technik auf Grund seines Aufbaus nicht für Unterschenkelknochen verwenden. Aus den FR 2 949 662 A1 und DE 103 17 776 A1 sind weitere Systeme zur Distraktion von Knochen bekannt.

### Aufgabe der Erfindung

Aufgabe der Erfindung ist es, einen Marknagel oder ein Baukastensystem für einen Marknagel anzugeben, wobei aus dem Stand der Technik bekannte Systeme oder Marknägel verbessert werden sollen, insbesondere sollen die Nachteile des Standes der Technik gelindert oder behoben werden. Wünschenswert sind Marknägel, welche einen möglichst geringen Operationsaufwand benötigen oder eine zuverlässige Fixierung in den Knochenfragmenten ermöglichen. Ein Baukastensystem sollte flexibel an unterschiedliche Gegebenheiten, beispielsweise verschiedene Knochen oder verschiedene Anwendungen wie Segmenttransport oder Distraktion, anpassbar sein.

Die Aufgabe wird mit einem Marknagel nach dem Anspruch 1 gelöst.

Beispielhafte Marknägel weisen einen sich in axialer Richtung des Marknagels erstreckenden hohlen Rumpf auf. Dieser Rumpf weist vorzugsweise axial ausgerichtete Langlöcher zur Durchführung von Segmentfixierungsbolzen auf. Durch das Anordnen von Langlöchern wird ermöglicht, dass mit einem Segmentfixierungsbolzen ein Knochensegment fixiert werden kann. Das Knochensegment ist dabei üblicherweise ein Teil des Knochens, welcher zwischen einem ersten Knochenfragment und einem zweiten Knochenfragment angeordnet ist. Bei Ausführungsformen kann das erste Knochenfragment ein proximales Knochenfragment sein oder ein distales

Knochenfragment. Dementsprechend ist das zweite Knochenfragment dann ein distales Knochenfragment oder im anderen Fall ein proximales Knochenfragment. Beispielhafte Marknägel eignen sich insbesondere zur Behandlung von Frakturen oder anderen Schädigungen von langen Röhrenknochen, wobei andere Schädigungen beispielsweise Knochenverluste aufgrund von Tumoren oder Gewalteinwirkung sein können. Knochen, welche mit typischen Marknägeln behandelt werden können, sind Oberschenkelknochen (Femur) und Schienenbein (Tibia), es können jedoch auch Oberarmknochen (Humerus), Elle (Ulna), Speiche (Radius) und Wadenbein (Fibula) behandelt werden. Typische hier beschriebene Marknägel sind besonders auch für kleinwüchsige oder minderjährige Patienten geeignet, da der Aufbau beispielhafter Marknägel der Erfindung eine vergleichsweise geringe Länge der Knochenfragmente zur Verriegelung benötigt.

Typische Marknägel der Erfindung weisen ein Verriegelungsmittel zum Verriegeln des Rumpfes in dem ersten Knochenfragment des Röhrenknochens auf. Auf diese Weise kann der Rumpf mit einem ersten Knochenfragment des Röhrenknochens in alle Richtungen und alle Drehrichtungen fixiert verbunden werden. Der Rumpf ist damit in allen Freiheitsgraden mit dem Knochenfragment fixiert verbunden. Als Verriegelungsmittel kommen insbesondere Bolzen oder Schrauben in Betracht, welche in radialer Richtung durch den Rumpf hindurch greifen. Die Schrauben oder Bolzen ermöglichen eine Verankerung des Rumpfes in dem Knochenfragment.

Typischerweise umfasst der Marknagel einen Antrieb zum axialen Verschieben eines ersten Innenteils. Das erste Innenteil ist innerhalb des Rumpfes in axialer Richtung verschiebbar angeordnet. Typische Ausführungsformen des Innenteils sind zumindest im Wesentlichen zylinderförmig. Das Innenteil ist vorzugsweise ausformungslos, d.h. es weist beispielsweise keine nach außen radial abstehenden Zapfen auf, oder weist lediglich Öffnungen auf. Das Innenteil weist vorzugsweise ein Innengewinde auf, welches sich zumindest über einen Teil der Innenmantelfläche nahe der Öffnung zur Aufnahme der Antriebsspindel erstreckt. Typischerweise ist der Rumpf ebenfalls zylinderförmig. Übliche Rumpfabmessungen weisen einen Durchmesser des Rumpfes über seine gesamte Länge oder zumindest im Wesentlichen über seine gesamte Länge unverändert von maximal 13 mm auf. Weitere typische Marknägel der Erfindung weisen Rumpfdurchmesser von maximal 12 mm oder maximal 11 mm auf. Ein geringer Durchmesser bietet Vorteile, da dadurch ein Einführen des Marknagels erleichtert wird gegebenenfalls und ein Aufbohren der Markhöhle nur bis zu einem geringen Grad notwendig wird.

Bevorzugte erste Innenteile des Marknagels weisen zumindest eine radiale Durchgangsöffnung zur Aufnahme eines Segmentbolzens zum Fixieren eines Knochensegments des Röhrenknochens auf. Dadurch, dass der Segmentfixierungsbolzen auch durch zwei gegenüber liegend angeordnete axial ausgerichtete Langlöcher des Rumpfes geführt werden kann, wird gleichzeitig das Innenteil gegenüber dem Rumpf bezüglich des Drehfreiheitsgrades um die Längsachse des Marknagels gesperrt. Weiterhin bietet dies den Vorteil, dass auch das Knochensegment sich während einer Behandlung nicht um die Längsachse des Marknagels verdreht.

Weitere Ausführungsformen umfassen einen Riegel zum Fixieren des Knochensegmentes. Ein solcher Riegel bietet den Vorteil eines einfachen Aufbaus. Der Riegel ist vorzugsweise quer zur Richtung einer Antriebsspindel ausgerichtet. Der Riegel bildet somit vorzugsweise einstückig sowohl das erste Innenteil als auch das Fixierungsmittel. Beispielhafte Ausführungsformen mit mehrteiligem Riegel umfassen ein Innenteil, typischerweise ähnlich einer Mutter, und einen radial abstehendes Riegelelement. Hierzu ist der Riegel typischerweise mit einer Ausnehmung versehen. Diese Ausnehmung greift vorzugsweise formschlüssig in ein auf der Spindel sitzendes Innenteil, welches als Gewindeelement mit einem mittig angeordneten Innengewinde ausgeführt ist, ein. Diese Ausführungsform erlaubt eine Reduzierung der Baulänge oder die Möglichkeit, größere Distraktionsstrecken zu realisieren.

Der Antrieb ist typischerweise fixiert in dem Rumpf aufgenommen, sodass ein Einführen des Marknagels in den Röhrenknochen mit aufgenommenen Antrieb ermöglicht wird. Bei typischen Ausführungsformen ist der Marknagel aufgebaut, sodass ein Einführen des Marknagels in den Röhrenknochen mit aufgenommenem Antrieb, das heißt mit in dem Rumpf aufgenommenem Antrieb, ermöglicht wird. Bevorzugte Marknägel ermöglichen eine Vorgehensweise mit vorherigem Einbau des Antriebs (vor Einsetzen in den Knochen) bei Operationen, wobei Fragmentfixierungsbolzen oder andere Verriegelungsmittel auch bei bereits eingesetztem Antrieb im Knochen und im Marknagel verankert werden können. In weiteren Ausführungsformen kann der Antrieb auch nachträglich während einer Operation in den Marknagel eingesetzt werden. Bei Ausführungsformen, insbesondere bei solchen mit nachträglich einsetzbarem Antrieb, sind typischerweise Mittel zur Verdrehsicherung vorgesehen.

Typische Marknägel umfassen ein zweites Innenteil, welches innerhalb des Rumpfes in axialer Richtung verschiebbar angeordnet ist. Durch Vorsehen eines ersten und eines zweiten Innenteils kann erreicht werden, dass eine Segmentverschiebung und außerdem noch eine Distraktion des gesamten Knochens, das heißt eine Verschiebung der beiden Knochenfragmente relativ zueinander erreicht werden kann. Insbesondere bei solchen Ausführungsformen ist das Ende des Rumpfes, welcher im Bereich des zweiten Innenteils ist, nach einem Einbau in einen Knochen in dem entsprechenden Knochenfragment verschiebbar. Das Knochenfragment wird typischerweise über Fragmentfixierungsmittel oder Fragmentfixierungsbolzen des zweiten Innenteils gehalten.

Die Innenteile sind üblicherweise axial hintereinander in dem Rumpf angeordnet. Bei Ausführungsformen sind die Innenteile unmittelbar benachbart oder können sogar aneinander anstoßen. Dies bietet den Vorteil, dass nach einem Segmenttransport anschließend eine unmittelbare Kraftübertragung von Innenteil zu Innenteil für eine Distraktion möglich ist. Bei weiteren Ausführungsformen ist ein Aneinanderstoßen der Innenteile nicht vorgesehen. Dies bietet den Vorteil, dass am Ende eines Segmenttransports bei Anstoßen des Knochensegments an einem der Knochenfragmente ein hoher Druck zwischen Segment und Knochenfragment ausgeübt werden kann, sodass ein Zusammenwachsen des Knochensegmentes mit dem Knochenfragment ermöglicht wird, auch während einer auf den Segmenttransport folgenden Distraktion. Dabei wird eine Vorschubkraft vom ersten Innenteil mit Knochensegment auf das Knochenfragment und das damit verbundene zweite Innenteil übertragen, welches dadurch im Rumpf nach hinten geschoben wird.

Üblicherweise erfolgt der Segmenttransport derart, dass das Knochensegment von einem Knochenfragment langsam in Richtung des anderen Knochenfragments verschoben wird, wobei in dem dadurch entstehenden Zwischenraum zwischen Ausgangs-Knochenfragment und Knochensegment Knochen nachwächst. Einzelheiten können der Patentschrift DE 197 08 279 C2 entnommen werden.

Vorzugsweise sind die beiden Innenteile axial hintereinander in dem Rumpf ohne feste Verbindung angeordnet. Die Innenteile sind üblicherweise lediglich in axialer Richtung verschiebbar. Bei typischen Marknägeln sind die Innenteile axial geführt. Das zweite Innenteil ist bei typischen Ausführungsformen unabhängig vom ersten Innenteil verschiebbar.

Vorzugsweise weist der Rumpf im Bereich des zweiten Innenteils weitere Langlöcher zur Durchführung von zumindest einem Fragmentfixierungsbolzen zum Fixieren eines zweiten Knochenfragmentes des Knochens auf. Typischerweise sind zwei in axialer Richtung des Rumpfes ausgerichtete Langlöcher gegenüberliegend in dem Rumpf zur Durchführung des Fragmentfixierungsbolzens oder der mehreren Fragmentfixierungsbolzen vorgesehen. Dabei bedeutet "im Bereich des zweiten Innenteils" beispielsweise einen Bereich, in welchem Durchgangsöffnungen des zweiten Innenteils liegen, wenn der zweite Innenteil im Einsatz wie vorgesehen verschoben wird.

Üblicherweise weist das zweite Innenteil eine in radialer Richtung ausgerichtete Durchgangsöffnung zur Aufnahme des Fragmentfixierungsbolzens auf. Bei typischen Ausführungsformen weist das Innenteil zwei oder mehr in radialer Richtung ausgerichtete Durchgangsöffnungen zur Aufnahme von Fragmentfixierungsbolzen auf. Dies bietet den Vorteil, dass das zweite Knochenfragment sicher verriegelt werden kann.

Typische Marknägel weisen ein antriebsloses zweites Innenteil auf. Dabei bedeutet "antriebslos", dass das zweite Innenteil nicht über einen eigenständigen Antrieb verfügt und nicht mit dem Antrieb verbunden ist, beispielsweise durch einen Spindeleingriff oder eine Schubstange. Bei Ausführungsformen ist das erste Innenteil fest mit einem Antrieb verbunden, wohingegen das zweite Innenteil frei verschiebbar ist. Dabei wird der Verschiebeweg des zweiten Innenteils vorzugsweise durch einen Anschlag oder durch das erste Innenteil begrenzt. Das zweite Innenteil wird im Betrieb bei Ausführungsformen erst verschoben, wenn das Knochensegment an dem zweiten Knochenfragment anstößt oder wenn das erste Innenteil unmittelbar an das zweite Innenteil anstößt.

Vorzugsweise ist an einem Ende des Rumpfes eine Herzogkrümmung vorgesehen. Dies bietet die Möglichkeit, den Marknagel auch in einem Unterschenkelknochen (Tibia) einzusetzen. Vorzugsweise ist die Herzogkrümmung am Ende des Antriebs vorgesehen. Bei typischen Ausführungsformen ist der Antrieb gegenüberliegend zum zweiten Innenteil angeordnet, das heißt, dass das erste Innenteil zwischen dem zweiten Innenteil und dem Antrieb liegt. Vorzugsweise ist der Rumpf mit Ausnahme der Herzogkrümmung und der Öffnungen zur Durchführung von Bolzen oder Verriegelungsmitteln ideal zylindrisch. Dabei bedeutet "ideal zylindrisch", dass insbesondere keine sonstigen Stifte oder Nasen oder Längsöffnungen an dem Rumpf vorgesehen sind. Bei typischen Ausführungsformen ist der Marknagel derart ausgebildet, dass die Herzogkrümmung und der Antrieb proximal angeordnet sind. Typischerweise ist der Antrieb distal, d.h. hinter oder unterhalb, der Herzogkrümmung angeordnet.

Als Antrieb wird vorzugsweise ein elektrischer Motor mit Getriebe und Spindel vorgesehen. Das Getriebe ist vorzugsweise ein Planetengetriebe, da es hohe Übersetzungen ermöglicht. Zur Energieversorgung und Steuerung des Antriebs ist typischerweise eine Kontrolleinheit vorgesehen, welche drahtlos von außerhalb eines Körpers, in welchen der Marknagel eingesetzt ist, über eine Antenne mit Energie oder Steuersignalen versorgt werden kann. Bei Ausführungsformen ist die Kontrolleinheit dazu in der Lage, erfasste Größen, wie beispielsweise eine zur Verschiebung erforderliche Kraft oder einen bereits zurückgelegten Verschiebeweg drahtlos zu übermitteln oder zu senden. Bei weiteren Ausführungsformen ist als Antrieb eine Formgedächtnislegierung oder ein anderer Antrieb vorgesehen.

Typischerweise weisen das erste Innenteil und das zweite Innenteil eine Zylinderform auf. Bei üblichen Ausführungsformen ist der Durchmesser des ersten Innenteils und des zweiten Innenteils identisch. Bei bevorzugten Ausführungsformen weisen die Innenteile keine Ausformungen auf, die Innenteile sind also ausformungslos ausgebildet. Dies bietet den Vorteil einer einfachen Herstellung und eines sicheren Vermeidens von Verkantungen oder Ähnlichem. Es ist jedoch auch möglich, Nasen vorzusehen, um zusätzlich zu den Fixierungsbolzen die Lage der Innenteile in dem Rumpf vorzugeben. Der Ausdruck "Ausformungslos" bezieht sich dabei auf die äußere Begrenzungsfläche des jeweiligen Innenteils, insbesondere das erste Innenteil weist üblicherweise ein Innengewinde auf. Das erste Innenteil ist bevorzugt als Hülse ausgebildet, welche über ein Innengewinde zum Eingriff mit der Spindel verfügt. Das zweite Innenteil ist bevorzugt als Zylinder ausgebildet. Das erste Innenteil wird durch das Spindelelement und den daran anschließenden Antrieb, positioniert im Antriebsrumpf, linear angetrieben.

Ein weiterer Aspekt betrifft ein Baukastensystem für Marknägel, insbesondere für Marknägel für eine Distraktion eines Röhrenknochens. Das Baukastensystem umfasst ein Verriegelungsrumpf mit einem Verriegelungsmittel zum Verriegeln des Verriegelungsrumpfes in einem ersten Knochenfragment des Röhrenknochens, ein sich in einer axialen Richtung erstreckenden hohlen Teilrumpf, in welchem ein erstes Innenteil angeordnet ist, welches innerhalb des Teilrumpfes in axialer Richtung verschiebbar angeordnet ist und welches eine in radialer Richtung ausgerichtete Durchgangsöffnung zur Aufnahme eines Bolzens umfasst. Der Bolzen ist typischerweise ein Fixierungsbolzen, insbesondere ein Segmentfixierungsbolzen oder ein Fragmentfixierungsbolzen zum Fixieren eines Knochensegmentes oder eines zweiten Knochenfragmentes. Weiterhin umfasst das Baukastensystem typischerweise einen Antriebsrumpf, welcher einen Antrieb umfasst, der in dem Antriebsrumpf angeordnet ist, zum axialen Verschieben eines ersten Innenteils. Der Verriegelungsrumpf, der Antriebsrumpf und der Teilrumpf sind in dieser Reihenfolge miteinander zu einem Marknagel verbindbar. Als Verbindungstechnik wird insbesondere Schweißen verwendet, es ist jedoch auch möglich, Schraubverbindungen oder andere Verbindungen vorzusehen. Schweißverbindungen haben den Vorteil, dass sie platzsparend sind. Vorzugsweise sind der Verriegelungsrumpf, der Antriebsrumpf und der Teilrumpf mit einem identischen oder in der Richtung der Aufzählung kleiner werdenden Durchmesser ausgeführt, sodass sich ein Marknagel schaffen lässt, der leicht einführbar ist. Insbesondere weist der Teilrumpf vorzugsweise zumindest einen gleich großen oder kleineren Durchmesser als der Antriebsrumpf auf. Der Verriegelungsrumpf oder der Teilrumpf sind vorzugsweise hohl ausgeführt. Ein hohl ausgeführter Verriegelungsrumpf ermöglicht die Durchführung einer Zuleitung oder einer Antenne oder die Aufnahme einer Kontrolleinheit. Die Verriegelungsmittel des Verriegelungsrumpfes umfassen typischerweise radial durchgehende Öffnungen zur Aufnahme von Verriegelungsbolzen oder Fragmentfixierungsbolzen zum Fixieren des ersten Knochenfragmentes gegenüber dem Verriegelungsrumpf. Grundsätzlich sind die oben im Zusammenhang mit dem Marknagel beschriebenen Merkmale auch bei dem Baukastensystem vorteilhaft verwendbar oder anwendbar.

Bei Ausführungsformen des Baukastensystems sind ein Teilrumpf mit zwei Paaren von gegenüberliegenden Langlöchern und zusätzlich ein Teilrumpf mit lediglich einem Paar von gegenüberliegenden Langlöchern vorgesehen. Bei letzterer Variante kann zusätzlich zumindest eine radiale Öffnung zur Rumpf-festen Montage von Bolzen vorgesehen sein oder auch keine weitere Öffnung.

Bei typischen Ausführungsformen sind zwei Varianten von Verriegelungsrümpfen vorgesehen, wobei eine erste Variante axial gerade und eine zweite Variante axial abgewinkelt ausgebildet ist. Die axial gerade Variante des Verriegelungsrumpfes eignet sich insbesondere für die Behandlung von Oberschenkelknochen (Femur), wohingegen sich die Variante des Verriegelungsrumpfes mit Herzogkrümmung insbesondere für eine Behandlung des Schienbeines (Tibia) eignet. Auf diese Weise wird mit identischen Teilen, wie Antriebsrumpf und Teilrumpf, ein Baukastensystem geschaffen, mit welchem mehrere verschiedene Marknägel herstellbar sind. Dies kann Produktionskosten reduzieren.

Vorteilhafterweise weist der Teilrumpf ein zweites axial verschiebbares Innenteil auf. Weiterhin umfasst der Teilrumpf entsprechende axial ausgerichtete Längsöffnungen, durch welche weitere Bolzen hindurchgeführt werden können. Auf diese Weise ist es möglich mit zwei verschiebbaren Innenteilen und entsprechenden Langlöchern in dem Teilrumpf eine Möglichkeit für einen Distraktions-Marknagel mit Segmentverschiebung zu schaffen.

Allgemein wird in dieser Anmeldung "axial" für eine Richtung entlang der Längsachse des Marknagels verwendet.

### Kurze Beschreibung der Zeichnungen

Nachfolgend werden Ausführungsformen anhand der beiliegenden Figuren näher erläutert, wobei die Figuren zeigen:
- Fig. 1: zeigt in einer schematischen Übersicht einen Marknagel mit Segmenttransport und anschließender Distraktion in einer Übersichtskizze;
- Fig. 2: zeigt einen Ausschnitt des Marknagels der Fig. 1 in einer Ausgangssituation in einer Schnittansicht;
- Fig. 3: zeigt den Marknagel der Fig. 1 nach erfolgtem Segmenttransport in einer Schnittansicht;
- Fig. 4: zeigt Teile des Marknagels der Fig. 1 in einer Schnittansicht nach zusätzlicher Disktraktion;
- Fig. 5: zeigt eine alternative Anordnung eines Marknagels in einem Knochen;
- Fig. 6: zeigt eine Anordnung eines Marknagels in einem anderen Knochen als bei den Fig. 1 bis 5;
- Fig. 7: zeigt einen aus einem Baukastensystem zusammengesetzten ersten Marknagel schematisch; und
- Fig. 8: zeigt einen aus demselben Baukastensystem zusammengesetzten weiteren Marknagel in einer schematischen Ansicht.

### Beschreibung von Ausführungsbeispielen

Nachfolgend werden anhand der Figuren Ausführungsbeispiele erläutert, wobei für gleiche oder ähnliche Teile gleiche Bezugszeichen verwendet werden. Gleiche oder ähnliche Teile werden unter Umständen nicht im Zusammenhang mit jeder Figur erneut erläutert.

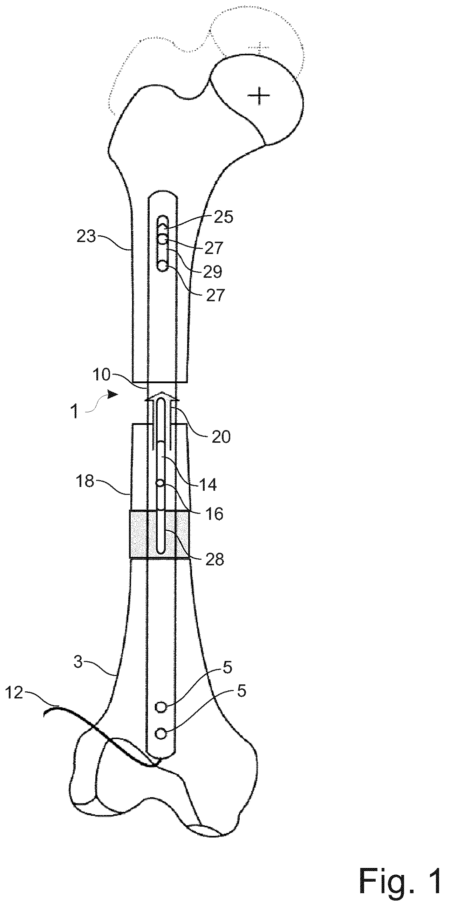

In der Fig. 1 ist ein Marknagel 1 dargestellt, welcher in einem ersten Knochenfragment 3 verriegelt ist. Der Marknagel 1 ist mit zwei Verriegelungsbolzen 5, welche als Verriegelungsmittel dienen, in dem ersten Knochenfragment 3 fixiert. Die Verriegelungsbolzen 5 sind im Wesentlichen parallel zur Sagittalebene ausgerichtet. Der Marknagel 1 umfasst einen Rumpf 10, in welchem Durchgangsöffnungen für die Verriegelungsbolzen 5 angeordnet sind. An dem Ende des Marknagels 1, welches in dem ersten Knochenfragment 3 verriegelt ist, ist eine Zuleitung 12 zur Versorgung des Marknagels mit Energie und Steuerdaten vorgesehen.

Der Rumpf 10 des Marknagels 1 ist teilweise hohl ausgeführt. In dem Rumpf 10 ist an dem Ende, an welchem die Zuleitung 12 befestigt ist, ein Antrieb (in Fig. 1 nicht gezeigt) in dem Marknagel 1 vorgesehen. Der Antrieb dient dazu, ein erstes Innenteil 14 anzutreiben. Das Innenteil 14 weist eine Durchgangsöffnung auf, in welchem ein Segmentbolzen 16 angeordnet ist. Der Segmentbolzen 16 fixiert ein Knochensegment 18 relativ zu dem ersten Innenteil 14. Durch Betätigen des Antriebs kann das erste Innenteil 14 von dem ersten Knochenfragment 3 weg verschoben werden. Die Verschieberichtung ist mit einem Pfeil 20 gekennzeichnet.

Bei Verschieben des Knochensegments 18 wächst bei ausreichend langsamer Vorschubgeschwindigkeit Knochen nach. Der nachwachsende Knochen entsteht an der Schnittstelle zwischen dem ersten Knochenfragment 3 und dem Knochensegment 18. Nach einem ausreichend weiten Vorschubweg stößt das Knochensegment 18 an einem zweiten Knochenfragment 23 an. In der Fig. 1 ist beispielhaft als erstes Knochenfragment ein distales Fragment eines Femur-Knochens (Oberschenkel) und als zweites Knochenfragment ein proximales Fragment des Femur-Knochens gezeigt.

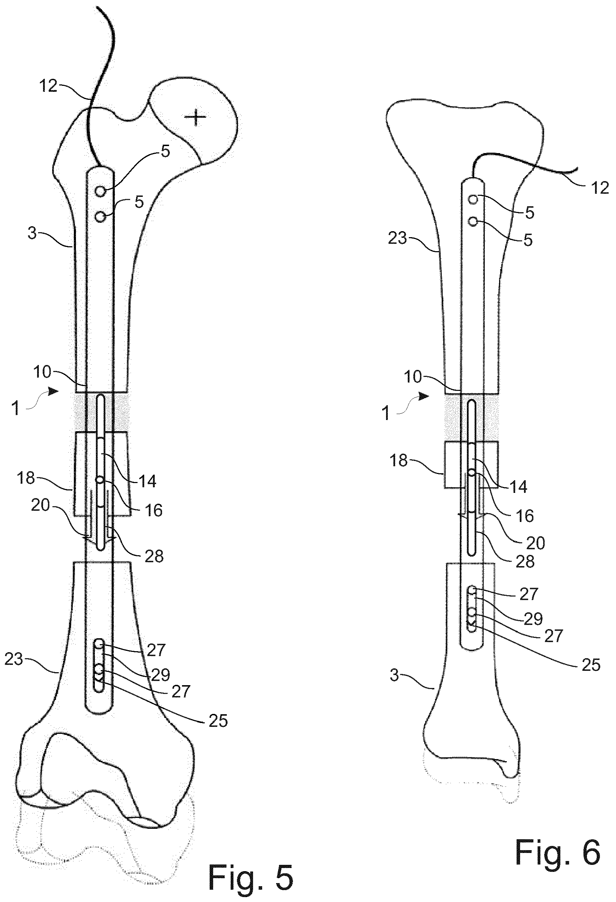

Je nach Größe der Knochenfragmente kann auch ein entgegen gesetzter Einbau des Marknagels sinnvoll sein, siehe Fig. 5. Bei weiteren Ausführungsformen ist der Antrieb nicht an dem verriegelungsseitigen Ende angeordnet, sondern an dem der Verriegelungsseite entgegen gesetzten Ende. Ein an dem Verriegelungsende angeordneter Antrieb drückt gegen das erste Innenteil beim Verschieben des ersten Innenteils, wohingegen ein an dem gegenüberliegenden Ende angeordneter Antrieb das Innenteil ziehend fortbewegt.

Nach Anstoßen des Knochensegments 18 an dem zweiten Knochenfragment 23 wird bei weiterem Vorschub in der Verschieberichtung 20 des ersten Innteils 14 auch ein Druck auf das zweite Knochenfragment 23 ausgeübt. Um ein Verschieben des zweiten Knochenfragments 23 relativ zu dem ersten Knochenfragment 3 zu ermöglichen, also um eine Distraktion zu ermöglichen, ist das zweite Knochenfragment 23 an einem relativ zu dem Rumpf 10 verschiebbaren zweiten Innenteil 25 angeordnet. Das zweite Knochenfragment 23 ist mit zwei Fragmentfixierungsbolzen 27 an dem zweiten Innenteil 25 befestigt. Die Fragmentfixierungsbolzen 27 sind identisch mit dem Segmentbolzen 16 und den Verriegelungsbolzen 5 ausgebildet.

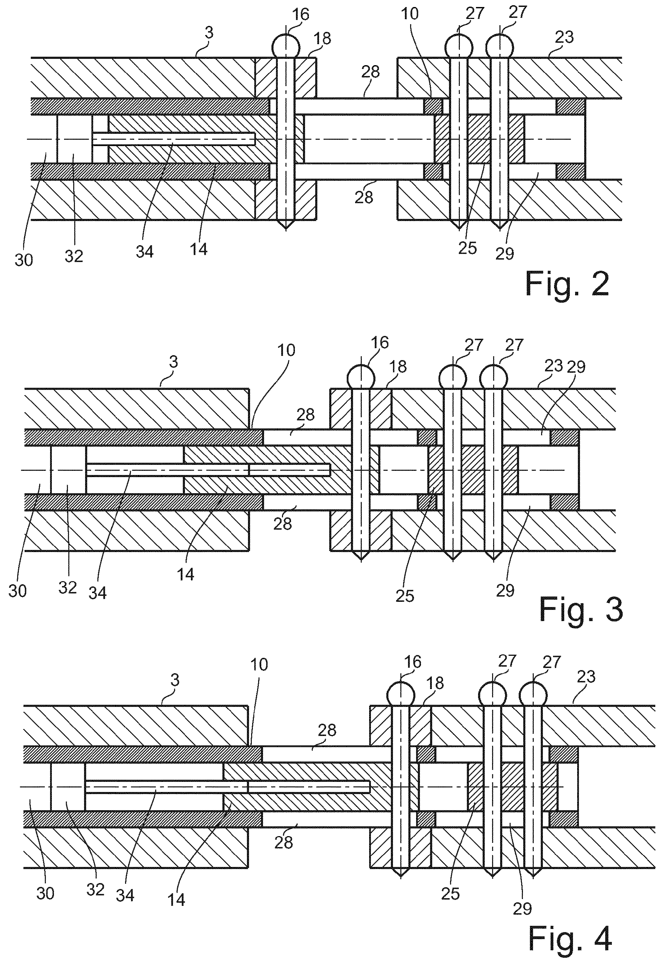

Alle Bolzen zum Fixieren sind in der Fig. 1 und in der Fig. 2 im Wesentlichen parallel zur Sagittalebene ausgerichtet. Typischerweise erfolgt eine Verriegelung jedoch senkrecht zur Sagittalebene. Dies bietet den Vorteil, dass ein Setzen der Bolzen oder Verriegelungsmittel vereinfacht ist. Eine Verriegelung ausschließlich in einer Richtung bietet den Vorteil, dass die Bolzen leichter gesetzt werden können, da sie alle in dieselbe Richtung gesetzt werden müssen.

Dadurch, dass der Vorschub des zweiten Knochenfragments 23 bei der Distraktion nach dem Segmenttransport des Knochensegments 18 über den Kontakt zwischen dem Knochensegment 18 und dem zweiten Knochenfragment 23 erfolgt, entsteht zwischen diesen beiden Knochenteilen ein Druck, welcher in der Regel ausreichend groß ist, um ein Zusammenwachsen des Knochensegments 18 mit dem zweiten Knochenfragment 23 zu unterstützen.

Bei dem Ausführungsbeispiel der Fig. 1 sind also das erste Innenteil und das zweite Innenteil derart ausgeführt, dass der Vorschub des zweiten Knochenfragments 23 nicht über einen Kontakt der Innenteile 14 und 25 erfolgt, sondern durch einen Kontakt des Knochensegments 18 mit dem zweiten Knochenfragment 23. Dies bietet den Vorteil, dass an der Kontaktfläche der Knochenteilstücke ein großer Druck aufgebaut werden kann. Alternativ ist es jedoch auch möglich, die Kraftübertragung zumindest teilweise direkt von einem Innenteil auf das andere Innenteil erfolgen zu lassen, beispielsweise auch mittels eines innerhalb des Rumpfes 10 angeordneten Zwischenteils zwischen den beiden Innenteilen 14 und 25.

In dem Rumpf 10 des Marknagels 1 sind für den Segmentbolzen 16 in axialer Richtung des Marknagels 1 ausgerichtete Langlöcher 28 vorgesehen, welche einen Durchtritt des Segmentbolzens 16 erlauben. Von den Langlöchern 28 ist lediglich eines dargestellt. Auf der Rückseite befindet sich ein weiteres Langloch 28. Für die Fragmentfixierungsbolzen 27 sind ebenso Langlöcher 29 an einem Ende des Rumpfes 10 des Marknagels 1 vorgesehen, wobei das Ende der Langlöcher 29 dem Ende mit den Verriegelungsbolzen 5 gegenüberliegt. Die Langlöcher 28 und 29 sind axial hintereinander angeordnet und ermöglichen eine Führung der Bolzen.

Typische Ausführungsformen weisen zwei gegenüberliegend angeordnete axial ausgerichtete Langlöcher im Bereich des Innenteils oder ersten Innenteils auf. Weitere Ausführungsformen weisen lediglich auf einer Seite ein Langloch auf. Daneben kann ein weiteres Langloch oder können weitere Langlöcher, insbesondere im Bereich eines zweiten Innenteils vorgesehen sein. Das mindestens eine weitere Langloch ist typischerweise gegenüber dem zumindest einen ersten Langloch axial verschoben, d.h. an einer in axialer Richtung des Marknagels anderen Stelle angeordnet.

Weiterhin ist in der Fig. 1 strichliert die Lage des zweiten Knochenfragments 23 nach erfolgter Distraktion dargestellt. Der Knochen kann mit Ausführungsbeispielen der Erfindung nach erfolgtem Segmenttransport noch um ein erhebliches Maß verlängert werden.

In den Fig. 2 bis 4 sind verschiedene Zustände des Marknagels 1 der Fig. 1 gezeigt, wobei in den Fig. 2 bis 4 lediglich ein Ausschnitt des Marknagels und des Knochens stark schematisiert dargestellt ist. So sind beispielsweise die Verriegelungsmittel zum Verriegeln des Marknagels 1 in dem ersten Knochenfragment 3 nicht dargestellt.

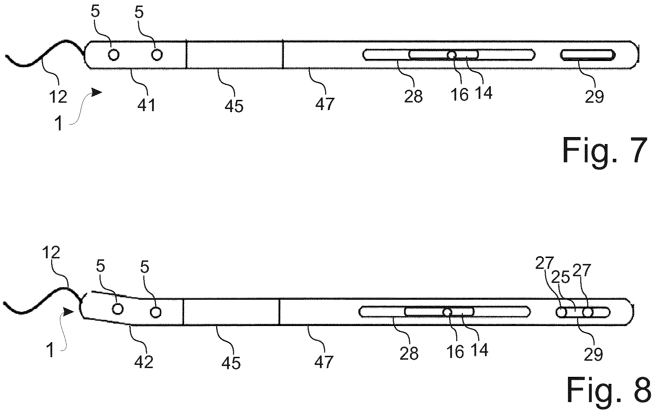

In der Fig. 3 ist auch ein Antriebsmotor 30 teilweise dargestellt. Der Antriebsmotor 30 ist in dem mehrteiligen Rumpf 10 aufgenommen. Die Mehrteiligkeit des Rumpfes 10 wird später im Zusammenhang mit den Fig. 7 und 8 genauer erläutert. Mit dem Motor 30 wird über ein Getriebe 32 eine Spindel 34 angetrieben, welche mit einem Innengewinde des ersten Innenteils 14 in Eingriff ist. Mit dem Antrieb ist es möglich, einen Segmenttransport des Knochensegments 18 durchzuführen, indem über die Spindel 34 das erste Innenteil 14 in Richtung des zweiten Innenteils 25 gedrückt wird.

Typische Ausführungsformen umfassen einen Motor als Antrieb. Weitere Antriebe, welche vorteilhaft verwendet werden können, sind durch externe Magnetfelder angetriebene Permanentmagnete, Piezoelemente, Formgedächtnislegierungen oder pneumatische Antriebe.

In der Fig. 3 sind außerdem die Langlöcher 28 noch einmal dargestellt, welche den Durchtritt des Segmentbolzens 16 (Langlöcher 28) und der Fragmentfixierungsbolzen 27 (Langlöcher 29) ermöglichen.

In der Fig. 3 ist der Marknagel in einem Zustand gezeigt, bei welchem das erste Innenteil 14 soweit verschoben ist, dass das Knochensegment 18 an dem zweiten Knochenfragment 23 anstößt. Wird nun der Motor 30 weiter betrieben und damit das erste Innenteil 14 weiter gedrückt, kommt es zu einer Verschiebung des zweiten Knochenfragmentes 23 in Richtung der Verschieberichtung des ersten Innenteils 14. Über das zweite Knochenfragment 23 wird auch das zweite Innenteil 25 in Richtung des Endes des Marknagels 1 verschoben. Dies bewirkt, dass nach dem Segmenttransport des Knochensegments 18 hin zu dem zweiten Knochenfragment 23 eine Distraktion erfolgt. Dies bedingt eine verschiebbare Anordnung des Rumpfs 10 im zweiten Knochenfragment 23. Die Länge des maximalen Knochenaufbaus des Marknagels der Fig. 3 bestimmt sich über die Länge des Langloches 28, wobei sich die Strecke des möglichen Segmenttransports aus der Länge des Langlochs 28 abzüglich der Länge des Langlochs 29 ergibt.

In der Fig. 4 ist der Zustand beim Abschluss der Distraktionsverschiebung gezeigt. Das zweite Innenteil 25 ist soweit verschoben, dass der endseitige der beiden Fragmentfixierungsbolzen 27 an dem Ende des Langlochs 29 des Rumpfes 10 anstößt. Es sollte angemerkt werden, dass in den Fig. 2 bis 4 nicht der nachgewachsene Knochen zwischen dem ersten Knochenfragment 3 und dem Knochensegment 18 dargestellt ist. Dies dient lediglich der Übersichtlichkeit.

Das Getriebe 32 ist ein Planetengetriebe und ermöglicht eine hohe Übersetzung. Weiterhin umfasst der Marknagel 1 eine Kontrolleinheit, welche über die Zuleitung 12 mit dem Marknagel 1 verbunden ist. Die Kontrolleinheit ermöglicht eine drahtlose Kommunikation von außerhalb des Körpers mit der Kontrolleinheit und damit ein drahtloses Steuern des Marknagels 1. Über die Kontrolleinheit kann auch der Motor 30 des Marknagels 1 mit Energie versorgt werden, wobei die Energieübertragung zu der Kontrolleinheit ebenso drahtlos erfolgt.

In der Fig. 5 ist ein gegenüber der Fig. 1 entgegen gesetzter Einbau des Marknagels 1 in einem Femur-Knochen gezeigt. Bei der Ausführungsform der Fig. 5 ist das erste Knochenfragment 3 das proximale Fragment des Knochens und das zweite Knochenfragment 23 das distale Knochenfragment des Knochens. Ansonsten wird analog auf die Beschreibung zur Fig. 1 verwiesen. Die Konfiguration der Fig. 5 kann insbesondere dann Vorteile bieten, wenn das distale Knochenfragment besonders klein ist.

In der Fig. 6 ist der Einbau eines Marknagels 1 in einem Tibia-Knochen gezeigt. Die Verriegelung des Marknagels 1 erfolgt in der Frontalebene oder auch Lateralebene. Es ist auch möglich, Marknägel für Tibia-Knochen mit einer Herzogkrümmung zu verwenden, wie dies beispielsweise in der Fig. 8 gezeigt ist. Weiterhin ist auch ein umgekehrter Einbau des Marknagels in einem Tibia-Knochen möglich, wobei bei einem umgekehrten Einbau die Zuleitung 12 vorzugsweise an dem Ende des zweiten Innenteils 25 angeordnet ist. Dies ermöglicht ein Einführen des Marknagels 1 in den Tibia-Knochen von der distalen Seite des Tibia-Knochens aus, wobei in diesem Fall auf die Herzogkrümmung verzichtet werden kann.

Die Fig. 7 und 8 zeigen verschiedene Marknägel 1, welche mit einem Baukastensystem gemäß Ausführungsformen zusammengebaut werden können.

Das Baukastensystem umfasst verschiedene Module, wobei für das Verriegelungsmodul zwei Varianten von Verriegelungsrümpfen vorgesehen sind, nämlich ein erster Verriegelungsrumpf 41, welcher gerade ausgebildet ist und ein zweiter Verriegelungsrumpf 42, welcher axial abgewinkelt ist. "Axial abgewinkelt" bedeutet, dass der Verriegelungsrumpf 42 in axialer Richtung des Marknagels 1 einen Winkel von wenigen Grad, beispielsweise zwischen 2° und 20° oder zwischen 3° und 15°, aufweist, eine so genannte Herzogkrümmung. Daher ist der Marknagel 1 der Fig. 8 mit der Herzogkrümmung des Verriegelungsrumpfes 42 besonders für Tibia-Knochen geeignet.

An die Verriegelungsrümpfe 41 und 42 ist jeweils ein Antriebsrumpf 45 angeschweißt, in welchem ein Antrieb, bspw. ein Motor, und ein Getriebe aufgenommen sind. An die Antriebsrümpfe 45 ist an dem anderen Ende wiederum jeweils ein Teilrumpf 47 angeschweißt, welcher axial ausgerichtete Langlöcher 28 und 29 aufweist. Die Antriebsrümpfe 45 und die Teilrümpfe 47 sind bei den Marknägeln der Fig. 7 und 8 identisch. Aus diesem Grund ist es mit einer begrenzten Teileanzahl möglich, verschiedene Marknägel herzustellen, wodurch Produktionskosten verringert werden können. Weiterhin ist es möglich, variabel ein Segmenttransport vorzusehen oder den Marknagel lediglich als Distraktionsmarknagel auszuführen. So umfasst der Marknagel 1 der Fig. 7 lediglich einen Segmentbolzen 16, welcher jedoch zur Fixierung eines zweiten Knochenfragmentes, also als Fragmentfixierungsbolzen verwendet wird. Das zweite Innenteil 25 wird dann nicht benötigt. Alternativ kann auch ein erstes Innenteil eingesetzt werden, welches dann ein zweites Innenteil unmittelbar durch Kontakt zwischen den Innenteilen verschiebt. Typischerweise bleiben alle Langlöcher, also erste Langlöcher 28 und zweite Langlöcher 29 zur Reduzierung der Teileanzahl auch bei Verwendung des Marknagels als reiner Distraktionsmarknagel erhalten. Wird jedoch eine längere Distraktionsstrecke benötigt oder muss der Marknagel aus anatomischen oder anderen Gründen im Vergleich zur Distraktionsstrecke verkürzt ausgebildet werden, so kann das Langloch 29 wegfallen und der Marknagel gekürzt werden.

Der Marknagel 1 der Fig. 8 umfasst wie die im Zusammenhang mit den Fig. 1 bis 6 beschriebenen Marknägel ein erstes Innenteil 14 zusammen mit einem Segmentbolzen 16, sodass mit dem Marknagel 1 der Fig. 8 auch ein Segmenttransport möglich ist. Daneben verfügt der Marknagel 1 der Fig. 8 auch über ein zweites Innenteil 25 für eine Distraktion.

Bei weiteren Ausführungsformen ist das zweite Innenteil verriegelbar. Dies bietet den Vorteil, dass bei Anwendungen, bei welchen keine Distraktionsstrecke benötigt wird, ein entsprechender Marknagel mit Segmenttransport durch einfaches Verriegeln des zweiten Innenteils geschaffen werden kann. Weitere Ausführungsformen eines Baukastensystems umfassen zusätzlich einen Teilrumpf, welcher lediglich über Langlöcher zur Aufnahme eines Fixierungsmittels für ein Knochensegment verfügt. Fixierungsmittel zur Fixierung des zweiten Knochenfragments sind bei solchen Ausführungsformen in radialen Öffnungen des Teilrumpfes aufgenommen, wobei eine Verschiebung der Fixierungsmittel für das zweite Knochensegment nicht möglich ist, beispielsweise durch feststehende radiale Öffnungen.

Der modulare Aufbau des Baukastensystems ermöglicht die Ausführung von verschiedenen Marknägeln mit einer begrenzten Teileanzahl. Über die gezeigten Beispiele hinaus sind weitere Möglichkeiten vorhanden, mit den gezeigten Bauteilen Marknägel zusammenzubauen. So ist es beispielsweise möglich, kürzere oder längere Teilrümpfe vorzusehen, um bei gleichem Antriebsrumpf Marknägel für unterschiedliche Knochenlängen herzustellen. Die Erfindung ist nicht auf die zuvor beschriebenen Ausführungsbeispiele beschränkt, vielmehr wird der Umfang der Erfindung durch die Ansprüche bestimmt. Insbesondere sind nicht alle dargestellten Teile notwendigerweise Merkmale der Erfindung, dies gilt in besonderem Maße für die dargestellten menschlichen Knochen.

### Bezugszeichen

- 1: Marknagel
- 3: Erstes Knochenfragment
- 5: Verriegelungsbolzen
- 10: Rumpf
- 12: Zuleitung
- 14: Erstes Innenteil
- 16: Segmentbolzen
- 18: Knochensegment
- 20: Verschieberichtung
- 23: Zweites Knochenfragment
- 25: Zweites Innenteil
- 27: Fragmentfixierungsbolzen
- 28, 29: Langlöcher
- 30: Motor
- 32: Getriebe
- 34: Spindel
- 41, 42: Verriegelungsrümpfe
- 45: Antriebsrumpf
- 47: Teilrumpf

## Patentansprüche

1. Marknagel (1) für eine Distraktion eines Röhrenknochens mit
- einem sich in einer axialen Richtung des Marknagels (1) erstreckenden zumindest teilweise hohlen Rumpf (10),
- einem Verriegelungsmittel zum Verriegeln des Rumpfes (10) in einem ersten Knochenfragment (3) des Röhrenknochens,
- einem ersten Innenteil (14), welches innerhalb des Rumpfes (10) in axialer Richtung verschiebbar angeordnet ist und ein Fixierungsmittel (16) zum Fixieren eines Knochensegments (18) des Röhrenknochens umfasst, wobei der Rumpf (10) im Bereich des ersten Innenteils (14) zumindest ein Langloch (28) zur Durchführung des Fixierungsmittels (16) umfasst,
- einem Antrieb zum axialen Verschieben des ersten Innenteils (14), und
- einem zweiten Innenteil (25), welches innerhalb des Rumpfes (10) in axialer Richtung verschiebbar angeordnet ist und ein Fragmentfixierungsmittel zum Fixieren eines zweiten Knochenfragments (23) des Röhrenknochens umfasst, und
**dadurch gekennzeichnet, dass**
das zweite Innenteil (25) antriebslos ist.

2. Marknagel (1) nach Anspruch 1, **dadurch gekennzeichnet, dass** der Rumpf (10) im Bereich des zweiten Innenteils (25) zumindest ein weiteres Langloch (29), insbesondere gegenüberliegende Langlöcher (29), zur Durchführung von zumindest einem Fragmentfixierungsbolzen (27) als Fragmentfixierungsmittel zum Fixieren eines zweiten Knochenfragmentes (23) des Röhrenknochens aufweist.

3. Marknagel (1) nach Anspruch 1 oder 2, **dadurch gekennzeichnet, dass** das zweite Innenteil (25) zumindest eine in radialer Richtung ausgerichtete Durchgangsöffnung zur Aufnahme des Fragmentfixierungsbolzens (27) umfasst.

4. Marknagel (1) nach einem der vorhergehenden Ansprüche, **dadurch gekennzeichnet, dass** das erste Innenteil (14) eine in radialer Richtung ausgerichtete Durchgangsöffnung zur Aufnahme eines Segmentbolzens (16) als Fixierungsmittel umfasst.

5. Marknagel (1) nach einem der vorhergehenden Ansprüche, **dadurch gekennzeichnet, dass** die Innenteile (14, 25) axial hintereinander in dem Rumpf (10) angeordnet sind.

6. Marknagel (1) nach einem der vorhergehenden Ansprüche, **dadurch gekennzeichnet, dass** der Antrieb fixiert in dem Rumpf (10) aufgenommen ist, so dass ein Einführen des Marknagels (1) in den Röhrenknochen mit aufgenommenem Antrieb möglich ist.

7. Marknagel (1) nach einem der vorhergehenden Ansprüche, **dadurch gekennzeichnet, dass** der Durchmesser des Rumpfes (10) über seine gesamte Länge unverändert ist und/oder maximal 13mm beträgt.

8. Marknagel (1) nach einem der vorhergehenden Ansprüche, **dadurch gekennzeichnet, dass** an einem Ende des Rumpfes (10) eine Herzogkrümmung vorgesehen ist.

9. Marknagel (1) nach einem der vorhergehenden Ansprüche, **dadurch gekennzeichnet, dass** der Antrieb in dem Rumpf (10) gegenüberliegend des zweiten Innenteils (25) angeordnet ist.

10. Marknagel (1) nach einem der vorhergehenden Ansprüche, **dadurch gekennzeichnet, dass** das erste Innenteil (14) und/oder das zweite Innenteil (25) eine Zylinderform aufweisen.

## Claims

1. An intramedullary nail (1) for distraction of a tubular bone including
- an at least partially hollow body (10) extending in the axial direction of the intermedullary nail (1),
- a locking means for locking the body (10) in a first bone fragment (3) of the tubular bone,
- a first inner portion, which is arranged within the body (10) so as to be movable in the axial direction, and includes fixing means (16) for fixing a bone segment (18) of the tubular bone, wherein the body (10) includes at least one elongate hole (28) for passing through the fixing means (16) in the region of the first inner portion (14),
- a drive for axially moving the first inner portion (14), and
- a second inner portion (25), which is arranged within the body (10) so as to be movable in the axial direction and includes a fragment fixing means for fixing a second bone fragment (23) of the tubular bone, and
**characterised in that** the second inner portion (25) is undriven.

2. An intramedullary nail (1) as claimed in Claim 1, **characterised in that** the body (10) has, in the region of the second inner portion (25), at least one further elongate hole (29), particularly opposing elongate holes (29), for passing through at least one fragment fixing peg (27) as the fragment fixing means for fixing a second bone fragment (23) of the tubular bone.

3. An intramedullary nail (1) as claimed in Claim 1 or 2, **characterised in that** the second inner portion (25) includes at least one opening, which is orientated in the radial direction, for receiving the fragment fixing peg (27).

4. An intramedullary nail (1) as claimed in one of the preceding claims, **characterised in that** the first inner portion (14) includes an opening, which is orientated in the radial direction, for receiving a segment peg (16) constituting the fixing means.

5. An intramedullary nail (1) as claimed in one of the preceding claims, **characterised in that** the inner portions (14, 25) are arranged axially behind one another within the body (10).

6. An intramedullary nail (1) as claimed in one of the preceding claims, **characterised in that** the drive is accommodated fixedly in the body (10) so that introduction of the intramedullary nail (1) into the tubular bone is possible with the drive accommodated.

7. An intramedullary nail (1) as claimed in one of the preceding claims, **characterised in that** the diameter of the body (10) is constant over its entire length and/or is at most 13mm.

8. An intramedullary nail (1) as claimed in one of the preceding claims, **characterised in that** a Herzog curvature is provided at one end of the body (10).

9. An intramedullary nail (1) as claimed in one of the preceding claims, **characterised in that** the drive is arranged within the body (10) opposite to the second inner portion (25).

10. An intramedullary nail (1) as claimed in one of the preceding claims, **characterised in that** the first inner portion (14) and/or the second inner portion (25) has a cylindrical shape.

## Revendications

1. Clou médullaire (1) pour une distraction d'un os long avec
- un corps (10) au moins partiellement creux s'étendant dans une direction axiale du clou médullaire (1),
- un moyen de verrouillage destiné à verrouiller le corps (10) dans un premier fragment d'os (3) de l'os long,
- une première partie intérieure (14) qui est disposée à l'intérieur du corps (10) de manière déplaçable dans la direction axiale et un moyen de fixation (16) destiné à fixer un segment d'os (18) de l'os long, le corps (10) comportant, à l'endroit de la première partie intérieure (14), au moins un trou oblong (28) destiné au passage du moyen de fixation (16),
- un entraînement pour le déplacement axial de la première partie intérieure (14), et
- une deuxième partie intérieure (25) qui est disposée à l'intérieur du corps (10) de manière déplaçable dans la direction axiale et un moyen de fixation de fragment destiné à fixer un deuxième fragment d'os (23) de l'os long, et
**caractérisé par le fait que**
la deuxième partie intérieure (25) est sans entraînement.

2. Clou médullaire (1) selon la revendication 1, **caractérisé par le fait que** le corps (10) présente, à l'endroit de la deuxième partie intérieure (25), au moins un autre trou oblong (29), en particulier des trous oblongs opposés (29), destiné au passage d'au moins un boulon de fixation de fragment (27) comme moyen de fixation de fragment pour fixer un deuxième fragment d'os (23) de l'os long.

3. Clou médullaire (1) selon la revendication 1 ou 2, **caractérisé par le fait que** la deuxième partie intérieure (25) comporte au moins une ouverture de passage orientée en direction radiale destinée à recevoir le boulon de fixation de fragment (27).

4. Clou médullaire (1) selon l'une des revendications précédentes, **caractérisé par le fait que** la première partie intérieure (14) comporte une ouverture de passage orientée en direction radiale destinée à recevoir un boulon de segment (16) comme moyen de fixation.

5. Clou médullaire (1) selon l'une des revendications précédentes, **caractérisé par le fait que** les parties intérieures (14, 25) sont disposées axialement l'une derrière l'autre dans la corps (10).

6. Clou médullaire (1) selon l'une des revendications précédentes, **caractérisé par le fait que** l'entraînement est reçu fixé dans le corps (10), de sorte qu'une introduction du clou médullaire (1) dans l'os long soit possible à entraînement emporté.

7. Clou médullaire (1) selon l'une des revendications précédentes, **caractérisé par le fait que** le diamètre du corps (10) est invariable sur toute sa longueur, et/ou est de maximum 13 mm.

8. Clou médullaire (1) selon l'une des revendications précédentes, **caractérisé par le fait qu'**à une extrémité du corps (10) est prévue une courbure de Herzog.

9. Clou médullaire (1) selon l'une des revendications précédentes, **caractérisé par le fait que** l'entraînement est disposé dans le corps (10), opposé à la deuxième partie intérieure (25).

10. Clou médullaire (1) selon l'une des revendications précédentes, **caractérisé par le fait que** la première partie intérieure (14) et/ou la deuxième partie intérieure (25) présentent une forme cylindrique.
